# EUROPÄISCHE PATENTSCHRIFT

(11) **EP 1 087 796 B1**
(45) Veröffentlichungstag und Bekanntmachung des Hinweises auf die Patenterteilung: **15.05.2002**
(21) Anmeldenummer: 99939353.1
(22) Anmeldetag: 16.06.1999
(51) Int. Cl.: A61L 11/00

(54) **VERFAHREN ZUR BEHANDLUNG DER FLÜSSIGEN PHASE VON KONTAMINIERTEN MATERIALIEN**
METHOD FOR TREATING THE LIQUID PHASE OF CONTAMINATED MATERIALS
PROCEDE DE TRAITEMENT DE LA PHASE LIQUIDE DE SUBSTANCES CONTAMINEES

(30) Priorität: 19.06.1998 DE 19827404
(43) Veröffentlichungstag der Anmeldung: 04.04.2001
(73) Patentinhaber: Göldner, Helmut, 31633 Leese (DE)
(72) Erfinder: Göldner, Helmut, 31633 Leese (DE)
(74) Vertreter: Braun, Dieter, Dipl.-Ing.
(86) Internationale Anmeldenummer: DE9901774
(87) Internationale Veröffentlichungsnummer: WO9966963

(56) Entgegenhaltungen:
- EP-A- 0 672 426
- DE-A- 4 138 939
- US-A- 5 759 491

## Beschreibung

Die Erfindung bezieht sich auf ein Verfahren zur Behandlung der flüssigen Phase von kontaminierten, insbesondere infizierten Materialien, die über eine Eingabeeinheit einem sich in einer Behandlungskammer einer Hochtemperaturdesinfektionsvorrichtung erstreckenden Transportsystem zugeführt, aufgeheizt und desinfiziert bzw. sterilisiert sowie an einem Auswurf ausgetragen werden, wobei ein definierter Sammelbereich für Flüssigkeit am Zufuhrende des Transportsystems in der Behandlungskammer vorgesehen ist.

Ferner betrifft die Erfindung eine Vorrichtung zur Behandlung von kontaminierten, insbesondere von infizierten Materialien, bei der diese über eine Eingabeeinheit einem sich in einer Behandlungskammer erstreckenden Transportsystem zugeführt, aufgeheizt und desinfiziert bzw. sterilisiert sowie an einem Auswurf ausgetragen werden, wobei ein definierter Sammelbereich für Flüssigkeit am Zufuhrende des Transportsystems in der Behandlungskammer vorgesehen ist.

In der DE 197 17 839 ist bereits eine Hochtemperaturdesinfektions- bzw. Hochtemperatursterilisationsvorrichtung beschrieben, die insbesondere für krankenhausspezifische Abfälle geeignet ist und bei der die Abfälle über einen Einlaßtrichter und einen Zerkleinerer zwei hintereinander angeordneten Förderschnecken-Streckenabschnitten zugeführt werden. Dabei ist die erste Förderschnecke als Aufheizschnecke ausgebildet. Diese ist in ihrer Förderrichtung schräg nach oben geneigt angeordnet, wodurch sicher erreicht wird, daß kontaminierte Flüssigkeit, die durch den Einwurftrichter eingeführt wird, sich nur in dem Bereich unterhalb des Einwurfstrichters bzw. in einem tiefgelegenen Förderschneckenabschnitt ansammeln kann.

Solange ein bestimmtes, in einem durchschnittlichen Rahmen liegendes Mischungsverhältnis von feuchtem und trockenem zu behandelndem Abfall eingehalten wird, bleibt das Flüssigkeitsniveau in dem unteren Teil der Aufheizschnecke unterhalb der zulassigen Höchstmarke Wenn jedoch große Mengen Abfall mit sehr hohem Flussigkeitsanteil in die Behandlungsanlage eingeführt werden, kann es passieren, daß das Flüssigkeitsniveau über die zulässige Höchstmarke im besagten Bereich ansteigt. Denn Abfall in rein flüssiger Phase kann aus prinzipiellen Gründen durch die Förderschnecken nicht befördert werden

Durch die US-A-5 759 491 wird ein Verfahren zur Behandlung von infizierten Materialien offenbart, bei dem das zu behandelnde Material über einen Sammelbereich einem Transportsystem zugeführt wird, welches sich durch die Behandiungskammer erstreckt. Dort findet die Aufheizung sowie Sterilisation bzw. Desinfektion statt. Der behandelte Schlamm wird am Austrag abgeführt. Aus einem Sammelbehälter kann Flüssigkeit in den Sammelbereich gepumpt oder diesem entnommen und in den Sammalbehälter gefördert werden.

Die EP-A-0 672 426 zeigt eine Vorrichtung zur Behandlung von infiziertem Abfall. Dabei wird dem zunächst zerschredderten Abfall in einer Verflüssigungsstation desinfizierte Flüssigkeit zugeführt. Diese Flüssigkeit wird durch eine an eine Granulier- und eine Desinfektionsstation anschließende Entwässerungsstation bereitgestellt. Hierdurch wird eine Rückführung der desinfizierten Flüssigkeit erreicht.

Die DE 41 38 939 zeigt schließlich eine Vorrichtung und ein Verfahren zum Sterilisieren und Desinfizieren von kontaminiertem Krankenhausmüll. Der granulierte Müll wird danach einer Desinfektionsschnecke zugeführt und getrocknet. Anschließend erfolgt eine Behandlung mit Sattdampf und einer emeuten Trocknung.

Der Erfindung liegt deshalb die Aufgabe zugrunde, ein Verfahren zur Verfügung zu stellen, mit dem auch große, in kurzer Zeit anfallende Mengen rein flüssigen Abfalls unabhängig von der eigentlichen für festes Abfallmaterial vorgesehenen Behandlungsstrecke einer Hochtemperaturdesinfektionsvorrichtung sicher behandelt werden können. Ferner liegt der Erfindung die Aufgabe zugrunde, zur Behandlung von kontaminierten, insbesondere von infizierten Materialien, die eine flüssige und eine feste Phase aufweisen, eine Vorrichtung vorzusehen, die auch große, in kurzer Zeit anfallende Mengen rein flüssigen Abfalls sicher zu desinfizieren bzw sterilisieren erlaubt.

Die Aufgabe wird in bezug auf das Verfahren durch ein gattungsgemäßes Verfahren gelöst, bei dem
- Flüssigkeit, die sich im definierten Sammelbereich der Behandlungskammer angesammelt hat, bei Erreichen eines vorgegebenen Maximal-Flüssigkeitspegels in einen Sammelbehälter abgeführt wird,
- wenn der Flüssigkeitspegel in der Behandlungskammer einen vorgegebenen Minimalwert erreicht, Flüssigkeit aus dem Sammelbehälter in die Behandlungskammer zurückgeführt wird.
- wenn der Flüssigkeitspegel in dem Sammelbehälter einen vorgegebenen Maximalwert erreicht, Flüssigkeit aus dem Sammelbehälter in die Behandlungskammer zurückgeführt wird. falls der Maximal-Flüssigkeitspegel in der Behandlungskammer nicht erreicht ist, und/oder in einer weiteren Hochtemperaturdesinfektionseinheit für reine Flüssigkeit behandelt wird.

Dadurch, daß Flüssigkeit aus dem definierten Sammelbereich der Behandlungskammer in einen Sammelbehälter abgeführt wird, wird erreicht, daß der vorgegebenen Maximal-Flussigkeitspegel in der Behandlungskammer nicht überschritten wird und sich damit kontaminierte Flüssigkeit nicht in unerwünschter Weise innerhalb der Behandlungskammer ausbreiten und zu einer Gefährdung des Behandlungsprozesses führen kann. Indem Flüssigkeit aus dem Sammelbehälter in die Behandlungskammer zurückgeführt wird, wenn in dieser Bedarf an Flüssigkeit besteht, ist andererseits erreicht, daß in der Behandiungskammer stets genügend Feuchtigkeit zur Verfügung steht, um den nötigen Dampfdruck zu erzeugen. Um zu verhindern, daß in dem Sammelbehälter ein vorgegebener Maximalwert des Flüssigkeitspegels überschritten wird, wird Flüssigkeit aus diesem Behälter wahlweise entweder in die Behandlungskammer zurückgeführt, solange dort der maximale Flüssigkeitspegel nicht erreicht ist. oder in einer zusätzlichen Hochtemperaturdesinfektionseinheit für reine Flüssigkeit behandelt.

Auf diese Weise ist erreicht, daß auch dann, wenn kontaminierte Materialien zu behandeln sind, die einen hohen Flüssigkeitsanteil aufweisen, eine sichere Desinfektion bzw. Sterilisation des Feststoffanteils des zu behandelnden Materials stets gewährleistet ist, indem sichergestellt ist, daß sich kontaminierte Flüssigkeit in der Behandlungskammer nicht unkontrolliert ausbreiten kann. Dadurch, daß Flüssiganteil zu behandelnden Materials einer zusätzlichen Hochtemperaturdesinfektionseinheit bzw. -sterilisationseinheit zugeführt wird, ist ferner erreicht, daß der behandelte Flüssigabfall z B. direkt in eine Abwasserleitung gefördert werden kann.

Vorzugsweise ist bei dem Verfahren vorgesehen, daß Flüssigkeit aus dem Sammelbehälter in die Behandlungskammer nur dann zurückgeführt wird, wenn der Flüssigkeitspegel in der Behandlungskammer einen vorgegebenen Minimalwert erreicht hat. Das bedeutet, daß, wenn der Flüssigkeitspegel in dem Sammelbehälter den vorgegebenen Maximalwert erreicht hat, im Regelfall Flüssigkeit aus dem Sammelbehälter in der zusätzlichen Hochtemperaturdesinfektionseinheit bzw. -sterilisationseinheit für reine Flüssigkeit behandelt wird.

Die Hochtemperaturdesinfektionseinheit kann im wesentlichen aus einem Wärmetauscher bestehen, in dem die zu behandelnde Flüssigkeit mindestens auf eine Temperatur aufgeheizt und diese Temperatur so lange gehalten wird, wie es für eine Desinfektion bzw. für eine Sterilisation erforderlich ist.

Die Desinfektion bzw. Sterilisation mittels des Wärmetauschers kann kontinuierlich erfolgen, indem ein permanenter Überdruck im Wärmetauscher dadurch erzeugt wird, daß der den Wärmetauscher verlassende Strom behandelter Flüssigkeit über ein Drosselorgan derartig reduziert wird, daß bei Einförderung mittels einer Pumpe sich in dem Wärmetauscher ein Druck aufbaut.

Wenn ein Wärmetauscher als zusätzliche Hochtemperaturdesinfektionseinheit vorhanden ist und Flüssigkeit aus dem Sammelbehälter in die Behandlungskammer nur dann zuruckgefuhrt wird, wenn der Flüssigkeitspegel in der Behandlungskammer einen vorgegebenen Minimalwert erreicht, - also Flüssigkeit aus dem Sammelbehälter sonst bei Erreichen eines Maximalwertes durch den Wärmetauscher hindurchgeführt wird -, ist vorzugsweise vorgesehen, daß der Sammelbehälter über den Wärmetauscher nur bis zu einem vorgegebenen Flüssigkeitspegel-Mittelwert entleert wird. Auf diese Weise wird erreicht, daß stets genügend Flüssigkeit in dem Sammelbehälter verbleibt, um den Flüssigkeitspegel in der Behandlungskammer bei Erreichen des vorgegebenen Minimalwertes ohne Frischwasserzufuhr erhöhen zu können.

Ferner kann erfindungsgemäß vorgesehen sein, daß in dem Sammelbehälter die Flüssigkeit desinfiziert oder sterilisiert werden kann, also die zusätzliche Hochtemperaturdesinfektionseinheit durch den Sammelbehälter selbst gegeben ist, und daß in diesem Fall weitere unbehandelte Flüssigkeit in einem Pufferbehälter aufgefangen werden kann, der zwischen der Behandlungskammer und dem Sammelbehälter angeordnet ist. In diesem Fall kann auf den nachgeschalteten Wärmetauscher verzichtet werden.

Es kann vorgesehen sein. daß der definierte Sammelbereich für Flüssigkeit der Behandlungskammer dadurch geschaffen ist, daß die Behandlungskammer in Förderrichtung aufwärts gerichtet geneigt ist. In diesem Fall sammelt sich in die Behandlungskammer eingeführte Flüssigkeit in einem unteren, durch die Neigung vorgegebenen Bereich der Behandlungskammer. Ferner basiert das Transportsystem vorzugsweise auf einer oder mehreren Förderschnecken.

Die Aufgabe der Erfindung wird in bezug auf die Vorrichtung durch eine gattungsgemäße Vorrichtung gelöst, die zusätzlich das Merkmal aufweist, daß der Sammelbereich der Behandlungskammer über mindestens eine Flüssigkeitsleitung mit einem Flüssigkeitssammelbehälter derartig verbunden ist, daß Flüssigkeit aus dem Sammelbereich der Behandlungskammer in den Sammelbehälter und aus diesem zurück in die Behandlungskammer geführt werden kann.

Auf diese Weise ist erreicht, daß sehr flexibel der Flüssigkeitspegel in dem Sammelbereich der Behandlungskammer verändert werden kann. So kann, wenn ein Maximalwert erreicht ist. Flüssigkeit aus der Behandlungskammer in den Flüssigkeitssammelbehälter geführt werden, um die Flüssigkeitsmenge in der Behandlungskammer zu reduzieren. Andererseits kann auch, wenn der Flüssigkeitspegel in der Behandlungskammer einen Minimalwert erreicht, Flüssigkeit aus dem Sammelbehälter in die Behandlungskammer zurückgeführt werden, um sicherzustellen, daß in dieser ausreichend Flüssigkeit zur Erzeugung des nötigen Dampfdruckes vorhanden ist.

Vorzugsweise ist vorgesehen, daß der Flüssigkeitssammelbehälter mit einer zusätzlich zur Behandlungskammer vorhandenen Hochtemperaturdesinfektionseinheit bzw. -sterilisationseinheit über eine Flüssigkeitsleitung verbunden ist. Dadurch ist erreicht, daß jederzeit Flüssigkeit aus dem Flüssigkeitssammelbehälter zu dessen Leerung abgeführt werden kann, wobei diese Flüssigkeit unabhängig von dem eigentlichen Behandlungsvorgang in der Behandlungskammer desinfiziert bzw. sterilisiert werden kann. Die so behandelte Flüssigkeit kann direkt in eine Abwasserleitung gefördert werden.

Diese zusätzliche Hochtemperaturdesinfektionseinheit bzw. -sterilisationseinheit kann auf einem Wärmetauscher basieren.

Alternativ kann auch vorgesehen sein, daß der Flüssigkeitssammelbehälter selbst als die zusätzliche Hochtemperaturdesinfektionseinheit bzw. -sterilisationseinheit ausgelegt ist. Bei dieser Ausführungsform der Vorrichtung ist vorzugsweise ferner vorgesehen, daß zwischen der Behandlungskammer und dem Flüssigkeitssammelbehälter ein Pufferbehälter für weitere aus der Behandlungskammer abgeführte unbehandelte Flüssigkeit angeordnet ist. Aus diesem Pufferbehälter kann nach Bedarf Flüssigkeit in die Behandlungskammer zurückgeführt werden.

Sowohl der Flüssigkeitssammelbehälter als auch der Pufferbehälter können belüftbar sein oder nach dem Prinzip eines Windkessels arbeiten. Während im ersteren Fall! eine Pumpe notwendig ist. um Flüssigkeit aus dem Sammelbehälter abzuführen, wird bei dem nach dem Prinzip eines Windkessels arbeitenden Flüssigkeitssammelbehälter durch Zuführung von Flüssigkeit in diesem ein Überdruck aufgebaut, der bei Öffnen eines Auslasses des Flüssigkeitssammelbehälters Flüssigkeit aus ihm austreibt. Auch bei einem Flussigkeitssammeibehälter, der auf dem Prinzip eines Windkessels basiert, kann zusätzlich eine Belüftungsmöglichkeit vorgesehen sein, um eine vollständige Entleerung des Flüssigkeitssammelbehälters zu erleichtern. Entsprechendes gilt für den Pufferbehälter.

Vorzugsweise ist die Behandlungskammer in Förderrichtung aufwärts gerichtet, um auf diese Weise den definierten Sammelbereich für Flüssigkeit in der Behandlungskammer zu erzeugen. Ferner ist vorzugsweise vorgesehen, daß das Transportsystem eine oder mehrere Förderschnecken aufweist, die sich innerhalb der Behandlungskammer erstrecken.

Im folgenden wird die Erfindung anhand zweier Ausführungsbeispiele der erfindungsgemäßen Vorrichtung näher erläutert, wobei auf die Figuren Bezug genommen wird. Es zeigen:
Figur 1, eine schematische Teildarstellung einer ersten erfindungsgemäßen Vorrichtung;
Figur 2, eine schematische Teildarstellung einer zweiten erfindungsgemäßen Vorrichtung.

In den beiden Figuren sind gleiche Teile mit gleichen Bezugszeichen bezeichnet.

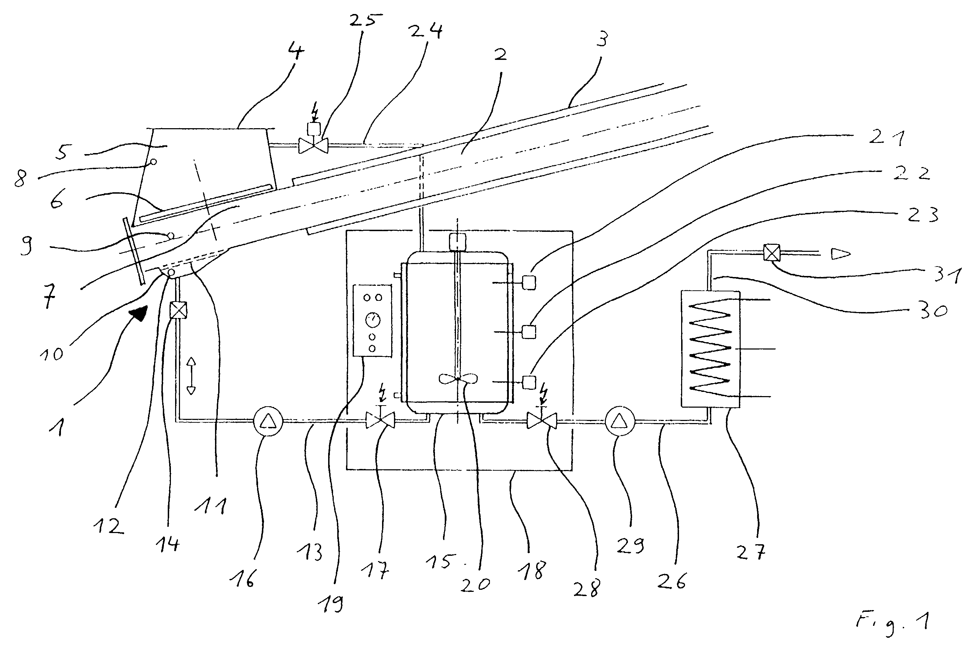

Die Vorrichtung gemäß Figur 1 - bezeichnet mit dem Bezugszeichen 1 - weist eine Behandlungskammer 2 (nur teilweise dargestellt) auf. Im wesentlichen über die gesamte Länge eines ersten Abschnitts der Behandlungskammer 2 erstreckt sich eine nicht gezeigte Förderschnecke. Die Aufheizschnecke ist über den größten Teil ihrer Länge mit einem Doppelmantel 3 versehen, in dem sich Wärmeträgeröl befindet, das in einem nicht gezeigten Aufheizblock erwärmt wird.

Die Vorrichtung 1 weist ferner eine Eingabeeinheit 4 mit einem Unterfalltrichter 5 und einem Dosierer 6 auf. Unterhalb der Eingabeeinheit 4, nämlich am Zufuhrende des durch die Aufheizschnecke gegebenen Transportsystems, befindet sich ein definierter Sammelbereich 7 für Flüssigkeit, die zusammen mit kontaminierten Feststoffen in den Unterfalltrichter 5 gegeben werden. Der definierte Sammelbereich 7 ergibt sich einerseits aus der vorgegebenen Neigung der Behandlungskammer 2 und andererseits aus einem Flüssigkeitsmelder 8, der einen vorgegebenen Maximal-Flüssigkeitspegel in der Behandlungskammer 2 festlegt. Ein weiterer Flüssigkeitsmelder 9, der innerhalb der Behandlungskammer 2 angeordnet ist, gibt einen Minimal-Flüssigkeitspegel in der Behandlungskammer 2 vor.

Ein Flüssigkeitsablaß 10 der Behandlungskammer 2 befindet sich unterhalb des Flüssigkeitsmelders 9. Der Flüssigkeitsablaß 10 ist durch ein Sieb 11 von der Aufheizschnecke getrennt. Innerhalb des Flüssigkeitsablasses 10 ist ein weiterer Flüssigkeitsmelder 12 angeordnet, der ein Signal liefert, wenn überhaupt keine Flüssigkeit mehr in der Behandlungskammer 2 vorhanden ist.

Eine Rohrleitung 13 ist über einen Anschluß 14 mit dem Flüssigkeitsablaß 10 verbunden und führt zu einem Sammelbehälter 15 für Flüssigkeit. Innerhalb der Rohrleitung 13 befinden sich eine Verdrängerpumpe 16 und ein elektrisches Kugelventil 17.

Der Sammelbehälter 15 ist in einem Gehäuse 18 angeordnet, der einen Schaltkasten 19 aufweist Ferner ist der Sammelbehälter 15 mit einem Rührwerk 20 und drei Füllstandsmeldern 21 22 und 23 ausgestattet. Eine Belüftungsleitung 24 mit einem elektrischen Ventil 25 verbindet den Unterfalltrichter 5 und die Oberseite des Sammelbehälters 15.

Eine weitere Rohrleitung 26 führt von der Unterseite des Sammelbehälters 15 zu einem Wärmetauscher 27. In der Rohrleitung 26 sind wiederum ein elektrisches Kugelventil 28 und eine Entladepumpe 29 angeordnet. Ausgangsseitig ist der Wärmetauscher 27 an eine weitere Rohrleitung 30 angeschlossen, die ein Drossel- und Absperrventil 31 aufweist und beispielsweise zu einer üblichen Abwasserleitung führt.

Der erfindungsgemäßen Vorrichtung 1 zur Behandlung von kontaminierten Materialien liegt folgende Funktionsweise zugrunde.

Kontaminiertes Material wird über die Eingabeeinheit 4 der Aufheizschnecke, die sich innerhalb des ersten Abschnitts der Behandlungskammer 2 erstreckt, zugeführt. Während der Beförderung durch die Aufheizschnecke wird das zu behandelnde Material definiert aufgeheizt. In der Aufheizschnecke erfolgt ferner eine Verdichtung des Materials in der Form, daß in deren Endbereich ein abdichtender Materialpfropfen erzeugt wird. Am Ende der Aufheizschnecke ist ein zweiter Abschnitt (nicht gezeigt) der Behandlungskammer angeordnet, in dem eine Entspannung und Auflockerung der Struktur des zu behandelnden Materials erfolgt. In dem zweiten Abschnitt der Behandlungskammer ist eine zweite Förderschnecke angeordnet, der das Material durch die Aufheizschnecke zugeführt wird. Die zweite Förderschnecke ist als von einem Heizelement umgebene Behandlungsschnecke für das Material ausgebildet. In den zweiten Abschnitt der Behandlungskammer und in die Behandlungsschnecke ist Energie stoßweise einleitbar, ferner kann Wasserdampf zugeführt oder/und erzeugt und ein Überdruck und die für die Desinfektion bzw. Sterilisation erforderliche Temperatur aufgebaut und gehalten werden. Die Behandlungsschnecke verdichtet das Material in ihrem Endbereich zu einem zweiten abdichtenden Materialpfropfen, derart, daß zwischen den beiden als Dichtung wirkenden Materialpfropfen der Schnecken der Überdruck für einen definierten Zeitraum gehalten werden kann. Am Ende der Behandlungsschnecke befindet sich ein Auswurf für das behandelte Material.

Durch die Neigung der Aufheizschnecke sammelt sich kontaminierte Flüssigkeit, die zusammen mit Feststoffmaterialien in die Eingabeeinheit 4 gegeben wird, in dem definierten Sammelbereich 7 der Behandlungskammer 2. Aus diesem Flüssigkeitsreservoir wird zusammen mit dem Feststoffmaterial, das durch die Aufheizschnecke zur Behandlungsschnecke befördert wird, auch Flüssigkeit zu der eigentlichen Behandlungsschnecke transportiert und dort sterilisiert.

Es ist vorgesehen, daß der Flüssigkeitspegel in dem Sammelbereich 7 der Behandlungskammer 2 nicht unter den durch den Flüssigkeitsmelder 9 vorgegebenen Minimalwert absinkt. Andererseits soll gewährleistet sein, daß der Flüssigkeitspegel nicht den durch den Flüssigkeitsmelder 8 vorgegebenen Maximalwert überschreitet.

Wenn der Flüssigkeitsmelder 8 anzeigt, daß der Maximalwert des Flüssigkeitspegels in dem Sammelbereich 7 erreicht ist, wird das Kugelventil 17 geöffnet und durch die Verdrängerpumpe 16 Flüssigkeit aus dem Sammelbereich 7 in den Sammelbehälter 15 gefördert. Dies geschieht so lange, bis der Flüssigkeitspegel in dem Sammelbereich 7 den Minimalwert erreicht. Anschließend wird das Kugelventil 17 wieder geschlossen. Über die Belüftungsleitung wird der Sammelbehälter 15 belüftet.

Wenn durch Unterschreiten des Minimal-Flüssigkeitspegels in dem Sammelbereich 7 ein Bedarf an zusätzlicher Flüssigkeit in der Behandlungskammer entsteht, wird wiederum über die Rohrleitung 13 Flüssigkeit aus dem Sammelbehälter 15 in den Sammelbereich 7 mittels der reversibel betätigbaren Verdrängerpumpe 16 gefördert. Dies geschieht so lange, bis entweder der Maximal-Flüssigkeitspegel in dem Sammelbereich 7 erreicht ist oder bis der Flüssigkeitspegel in dem Sammelbehälter 15 auf den durch den Füllstandsmeider 23 vorgegebenen Füllstand abgesunken ist. Dieser minimale Füllstand ist notwendig, damit das zur Verhinderung einer Sedimentation vorgesehene Rührwerk 20 des Sammelbehälters 15 noch in Flüssigkeit eintaucht.

Wenn der Füllstand des Sammelbehälters 15 die durch den weiteren Füllstandsmelder 21 definierte Höhe infolge größeren Flüssigkeitsanfalls in der Behandlungskammer erreicht, wird das Kugelventil 28 geöffnet und über die Entladepumpe 29 Flüssigkeit in den Wärmetauscher 27 geführt. Dies geschieht jedoch nur so lange, bis in dem Sammelbehälter 15 der durch den Füllstandsmelder 22 vorgegebene Füllstand erreicht ist. Dadurch wird sichergestellt, daß stets genügend Flüssigkeit in dem Sammelbehälter 15 zur Rückführung in die Behandlungskammer 2 vorhanden ist.

Der Sterilisationsprozeß im Wärmetauscher 27 kann sowohl kontinuierlich, als auch diskontinuierlich erfolgen. Im diskontinuierlichen Betrieb wird das am Ende des Wärmetauschers 27 befindliche Absperrventil 31 geschlossen, und die von der Entladepumpe 29 unter Druck gesetzte Flüssigkeit in dem Wärmetauscher 27 verbleibt für die zur sicheren Sterilisation vorgesehene Mindestverweilzeit in dem Wärmetauscher. Anschließend wird das Absperrventil 31 geöffnet, und die sterilisierte Flüssigkeit wird aus dem Wärmetauscher 27 ausgefördert.

Im kontinuierlichen Betrieb wird das Ventil 31 als Drosselorgan eingesetzt, wobei mittels der Entladepumpe 29 permanent in den Wärmetauscher 27 Flüssigkeit aus dem Sammelbehälter 15 eingefördert und hinter dem Drosselventil 31 ausgefördert. Dieser kontinuierliche Betrieb wird allerdings ausgesetzt, wenn der Flüssigkeitsstand in dem Sammelbehälter 15 die durch den Füllstandsmelder 22 gegebene Höhe erreicht. Im kontinuierlichen Sterilisationsbetrieb durch den Wärmetauscher 27 sind die Parameter so eingestellt, daß jedes Flüssigkeitsteilchen vom Eintritt in den Wärmetauscher 27 bis zum Austritt hinter dem Drosselventil 31 ausreichend lange im Wärmetauscher 27 verbleibt. Zwischen der Entladepumpe 29 und dem Wärmetauscher 27 ist dabei ein Rückschlagventil (nicht gezeigt) vorgesehen, um Rückströmungen aus dem Wärmetauscher 27 bei stillstehender Entladepumpe 29 in den Sammelbehälter 15 sicher zu vermeiden.

Der Warmetauscher 27 kann in einem Parallelstrom von demselben Wärmeträgeröl beheizt werden, das zur Beheizung der Behandlungskammer 2 bzw. der Aufheizschnecke vorgesehen ist.

Alternativ zu der in Figur 1 dargestellten Ausführungsform des Sammelbehälters 15 kann auch ein Sammelbehälter verwendet werden, der auf dem Prinzip des Windkessels basiert. Bei solch einem Sammelbehälter wird durch das Einführen von Flüssigkeit ein Druck innerhalb des Sammelbehälters aufgebaut, der zum Ausfördern von Flüssigkeit genutzt wird. In diesem Fall kann über die Belüftungsleitung 24 dann belüftet werden, wenn eine schnelle vollständige Entleerung des Sammelbehälters vorgenommen werden soll. Auf die Belüftungsleitung 24 kann aber auch in diesem Fall prinzipiell verzichtet werden. Wenn der Sammelbehälter auf dem Windkesselprinzip arbeitet, kann zusätzlich noch eine mit dem Sammelbehälter verbundene Druckquelle vorgesehen sein, um eine zusätzliche Möglichkeit zur Überdruckerzeugung in dem Sammelbehälter zu schaffen.

Ferner kann anders als bei der in Figur 1 gezeigten Vorrichtung vorgesehen sein, daß die Rohrleitung 13 nur zur Zuführung von Flüssigkeit in den Sammelbehälter 15 verwendet wird und zur Rückführung von Flüssigkeit aus dem Sammelbehälter 15 in die Behandlungskammer 2 eine weitere Leitung vorgesehen ist. In diesem Fall kann die Pumpe 16 als Pumpe, die nur in einer Richtung arbeitet, und das Ventil 17 als Rückschlagventil ausgelegt sein.

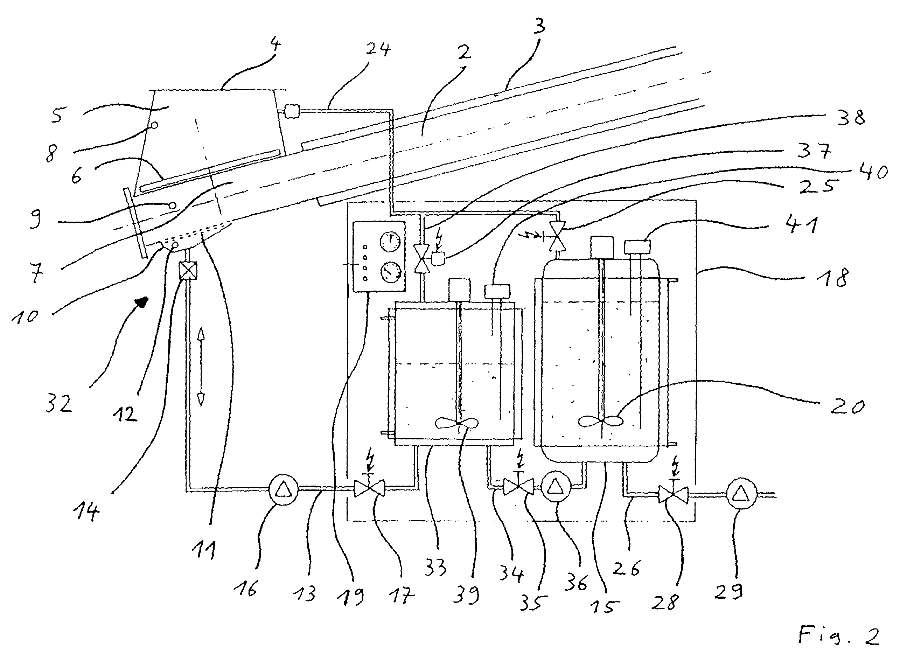

Im folgenden wird auf Figur 2 Bezug genommen. In dieser Figur ist eine weitere erfindungsgemäße Vorrichtung 32 gezeigt. Diese Vorrichtung 32 unterscheidet sich von der in Figur 1 dargestellten Vorrichtung 1 im wesentlichen dadurch, daß ein weiterer Behälter 33 vorgesehen ist, der zwischen der Behandlungskammer 2 und dem Sammelbehälter 15 angeordnet ist und als Pufferbehälter dient. Der Pufferbehälter 33 und der Sammelbehälter 15 sind über eine weitere Rohrleitung 34 miteinander verbunden. in dieser Rohrleitung 34 sind ein weiteres Kugelventil 35 und eine weitere Verdrängerpumpe 36 angeordnet.

Der Pufferbehälter 33 ist über eine ein Ventil 37 aufweisende Belüftungsleitung 38 mit der Belüftungsleitung 24 verbunden.

Der Pufferbehälter 33 weist ebenfalls ein Rührwerk 39 auf. Beide Behälter 33 und 15 sind jeweils mit einem Füllstandsmelder 40 bzw. 41 versehen, die jeweils einen minimalen Füllstand und einen maximalen Füllstand des Behälters vorgeben.

Bei der Vorrichtung 32 ist der Sammelbehälter 15 so ausgelegt, daß zusätzlich zu dem in Figur 1 gezeigten Wärmetauscher 27 in ihm die kontaminierte Flüssigkeit autoklaviert werden kann. Während dieses Sterilisationsprozesses kann weitere aus dem Sammelbereich 7 der Behandlungskammer 2 abzuführende Flüssigkeit in dem Pufferbehälter 33 aufgefangen werden, bzw. es kann Flüssigkeit aus dem Pufferbehälter 33 in die Behandlungskammer 2 zurückgeführt werden.

Bei dieser erfindungsgemäßen Vorrichtung 32 kann die in dem Sammelbehälter 15 behandelte Flüssigkeit direkt in einen Abwasserkanal geleitet werden. Ein weiterer Vorteil der Vorrichtung 32 liegt darin, daß zwei Behälter zum Auffangen von Flüssigkeit aus der Behandlungskammer 2 vorhanden sind, weshalb der einzelne Behälter kleiner ausgelegt sein kann.

Auch bei der Vorrichtung 32 können die beiden Behälter 35 und 15 nach dem Prinzip eines Windkessels arbeitend eingesetzt werden.

## Patentansprüche

1. Verfahren zur Behandlung der flüssigen Phase von kontaminierten, insbesondere infizierten Materialien, die über eine Eingabeeinheit (4) einem sich in einer Behandlungskammer (2) einer Hochtemperaturdesinfektionsvorrichtung erstreckenden Transportsystem zugeführt, aufgeheizt und desinfiziert bzw. sterilisiert sowie an einem Auswurf ausgetragen werden, wobei ein definierter Sammelbereich (7) für Flüssigkeit am Zufuhrende des Transportsystems in der Behandlungskammer (2) vorgesehen ist, **dadurch gekennzeichnet, daß**
- Flüssigkeit, die sich im definierten Sammelbereich (7) der Behandlungskammer (2) angesammelt hat, bei Erreichen eines vorgegebenen Maximal-Flüssigkeitspegels in einen Sammelbehälter (15) abgeführt wird,
- wenn der Flüssigkeitspegel in der Behandlungskammer (2) einen vorgegebenen Minimalwert erreicht, Flüssigkeit aus dem Sammelbehälter (15) in die Behandlungskammer (2) zurückgeführt wird,
- wenn der Flüssigkeitspegel in dem Sammelbehälter (15) einen vorgegebenen Maximalwert erreicht, Flüssigkeit aus dem Sammelbehälter (15) in die Behandlungskammer (2) zurückgeführt wird falls der Maximal-Flüssigkeitspegel in der Behandlungskammer (2) nicht erreicht ist, und/oder in einer weiteren Hochtemperaturdesinfektionseinheit (27) für reine Flüssigkeit behandelt wird.

2. Verfahren nach Anspruch 1, **dadurch gekennzeichnet, daß** die Flüssigkeit aus dem Sammelbehälter (15), die in der Hochtemperaturdesinfektionseinheit für reine Flüssigkeit zu behandeln ist, durch einen Wärmetauscher (27) hindurchgeführt wird, in dem sie mindestens auf eine Temperatur aufgeheizt und diese Temperatur so lange gehalten wird, wie es für eine Desinfektion oder Sterilisation erforderlich ist.

3. Verfahren nach Anspruch 2, **dadurch gekennzeichnet, daß** die Desinfektion bzw. Sterilisation mittels des Wärmetauschers (27) kontinuierlich erfolgt, indem ein permanenter Überdruck im Wärmetauscher (27) dadurch erzeugt wird, daß der den Wärmetauscher (27) verlassende Strom behandelter Flüssigkeit über ein Drosselorgan (31) beschränkt wird.

4. Verfahren nach Anspruch 2 oder 3, **dadurch gekennzeichnet, daß** in dem Fall, daß Flüssigkeit aus dem Sammelbehälter (15) in die Behandlungskammer (2) nur dann zurückgeführt wird, wenn der Flüssigkeitspegel in der Behandlungskammer (2) einen vorgegebenen Minimalwert erreicht, so lange Flüssigkeit aus dem Sammelbehälter (15) durch den Wärmetauscher (27) abgeführt wird, bis der Flüssigkeitspegel in dem Sammalbehälter (15) einen vorgegebenen Mittelwert erreicht hat.

5. Verfahren nach Anspruch 1, **dadurch gekennzeichnet, daß** die in der weiteren Hochtemperaturdesinfektionseinheit zu behandelnde Flüssigkeit in dem Sammelbehälter (15) desinfiziert oder sterilisiert wird und, insbesondere während eines Desinfektions- oder Sterilisationsvorgangs in dem Sammelbehälter (15), weitere unbehandelte Flüssigkeit in einem Pufferbehälter (33) aufgefangen wird, der zwischen der Behandlungskammer (2) und dem Sammelbehälter (15) angeordnet ist.

6. Verfahren nach einem der vorhergehenden Ansprüche, **dadurch gekennzeichnet, daß** der definierte Sammelbereich (7) für Flüssigkeit dadurch geschaffen wird, daß die Behandlungskammer (2) in Förderrichtung aufwärts gerichtet geneigt ist.

7. Verfahren nach einem der vorhergehenden Ansprüche, **dadurch gekennzeichnet, daß** das Tansportsystem eine Förderschnecke aufweist.

8. Vorrichtung zur Ausführung des Verfahrens nach den Ansprüchen 1-7 zur Behandlung von kontaminierten, insbesondere von infizierten Materialien, bei der diese über eine Eingabeeinheit (4) einem sich in einer Behandlungskammer (2) erstreckenden Transportsystem zugeführt, aufgeheizt und desinfiziert bzw. sterilisiert sowie an einem Auswurf ausgetragen werden, wobei ein definierter Sammelbereich (7) für Flüssigkeit am Zufuhrende des Transportsystems in der Behandlungskammer (2) vorgesehen ist, **dadurch gekennzeichnet, daß** der Sammelbereich (7) der Behandlungskammer (2) über mindestens eine Flüssigkeitsleitung (13) mit einem Flüssigkeitssammelbehälter (15) derartig verbunden ist, daß Flüssigkeit aus dem Sammelbereich (7) der Behandlungskammer (2) in den Sammelbehälter (15) und aus diesem zurück in die Behandlungskammer (2) geführt werden kann, wobei ein Flüssigkeitsmelder (8, 9) zur Erfassung des jeweiligen Füllstandes vorgesehen ist und daß der Flüssigkeitssammelbehälter (15) mit einer weiteren Hochtemperaturdesinfektionseinheit (27) über eine Flüssigkeitsleitung (26) verbunden ist.

9. Vorrichtung nach Anspruch 8, **dadurch gekennzeichnet, daß** die weitere Hochtemperaturdesinfektionseinheit einen Wärmetauscher (27) aufweist.

10. Vorrichtung nach Anspruch 9, **dadurch gekennzeichnet, daß** der Flüssigkeitssammelbehälter (15) als die weitere Hochtemperaturdesinfektionseinheit ausgelegt ist.

11. Vorrichtung nach Anspruch 10, **dadurch gekennzeichnet, daß** zwischen der Behandlungskammer (2) und dem Flüssigkeitssammelbehälter (15) ein Pufferbehälter (33) für die Flüssigkeit aus dem Sammelbereich (7) der Behandlungskammer (2) angeordnet ist.

12. Vorrichtung nach einem der Ansprüche 8 bis 11, **dadurch gekennzeichnet, daß** der Flüssigkeitssammelbehälter (15) nach dem Prinzip eines Windkessels arbeitet.

13. Vorrichtung nach einem der Ansprüche 8 bis 12, **dadurch gekennzeichnet, daß** die Behandlungskammer (2) in Förderrichtung aufwärts gerichtet ist.

14. Vorrichtung nach einem der Ansprüche 8 bis 13, **dadurch gekennzeichnet, daß** das Transportsystem eine Förderschnecke aufweist.

## Claims

1. A method for the treatment of the liquid phase of contaminated, especially infected, materials, which are taken via an input unit (4) into a treatment chamber of an extended transport system (2) of a high temperature disinfecting unit, heated and disinfected and taken to a discharge, whereby a defined collecting area (7) is provided for liquid at the infeed end of the transport system is provided in the treatment chamber (2), **characterised in that**
- liquid which has collected in the defined collection area (7) of the treatment chamber (2) is taken off to a collection container (15) on reaching a predetermined maximum liquid level,
- when the liquid in the treatment chamber (2) reaches a predetermined minimum level, liquid is taken back into the treatment chamber (2) from the collection container (15),
- when the liquid level in the collecting container (15) has reached a predetermined maximum value, liquid is taken back from the collection container (15) into the treatment chamber (2) providing that the maximum liquid level in the treatment chamber (2) is not reached, and/or is treated in a further high temperature disinfection unit (27) for liquid only.

2. A method according to Claim 1, **characterised in that** the liquid from the collection container (15), which is to be treated in the high temperature disinfection unit for liquid only, is taken through a heat exchanger (27) in which it is heated at least to a temperature and held at this temperature for as long as is necessary for disinfection or sterilisation.

3. A method according to Claim 2, **characterised in that** the disinfection or sterilisation occurs continuously by means of the heat exchanger (27) whilst a permanent pressure is created in the heat exchanger (27) **in that** the stream of treated liquid leaving the heat exchanger (27) is limited by a throttle element (31).

4. A method according to Claim 2 or Claim 3, **characterised in that** in the case that liquid is only then taken back from the collection container (15) into the treatment chamber (2) when the liquid level in the treatment chamber (2) reaches a prescribed minimum value, as long as liquid is taken out of the collection container (15) through the heat exchanger (27) until the liquid level in the collection container (15) has reached a prescribed middle value.

5. A method according to Claim 1, **characterised in that** the liquid to be treated in the further high temperature disinfection unit is disinfected or sterilised in the collection container (15) and especially during a disinfection or sterilisation process in the collection container (15), further untreated liquid is captured in a buffer container (33), which is arranged between the treatment chamber (2) and the collection container (15).

6. A method according to one of the foregoing Claims, **characterised in that** the defined collection area (7) for liquid is produced **in that** the treatment chamber (2) is directed upwards in the conveying direction.

7. A method according to one of the foregoing Claims, **characterised in that** the transport system has a helical conveyor.

8. An arrangement for the performance of the method according to Claims 1 - 7 for the treatment of contaminated, especially infected, materials, in which they are taken via an input unit (4) into a treatment chamber (2) of an extended transport system of a high temperature disinfecting unit, heated and disinfected and taken to a discharge, whereby a defined collecting area (7) is provided for liquid at the infeed end of the transport system in the treatment chamber (2), **characterised in that** the collecting area (7) of the treatment chamber (2) is connected via at least one liquid feed line (13) to a liquid collection container (15) in such a manner that liquid from the collecting area (7) of the treatment chamber (2) can be taken into the collection container (15) and from this back into the treatment chamber (2), whereby a liquid sensor (8, 9) is provided to determine the individual degree of filling and that the liquid collection container (15) is connected with a further high temperature disinfection unit (27) via a liquid feed line (26).

9. An arrangement according to Claim 8, **characterised in that** the further high temperature disinfection unit has a heat exchanger (27).

10. An arrangement according to Claim 9, **characterised in that** the liquid collection container (15) is designed as the further high temperature disinfection unit.

11. An arrangement according to Claim 10, **characterised in that** a buffer container (33) is arranged between the treatment chamber (2) and the liquid collection container (15) for the liquid from the collecting area (7) of the treatment chamber (2).

12. An arrangement according to one of the Claims 8 to 11, **characterised in that** the liquid collection container (15) operates on the principle of a surge tank.

13. An arrangement according to one of the Claims 8 to 12, **characterised in that** the treatment chamber (2) is directed upwards in the direction of conveying.

14. An arrangement according to one of the Claims 8 to 13, **characterised in that** the transport system has conveying helix.

## Revendications

1. Procédé de traitement de la phase liquide de substances contaminées, notamment infectées, qui sont amenées par l'intermédiaire d'une unité d'entrée (4) à un système de transport s'étendant dans une chambre de traitement (2) d'un dispositif de désinfection à haute température, chauffées et désinfectées ou stérilisées, ainsi qu'évacuées au niveau d'une sortie, une zone de collecte définie (7) pour du liquide étant prévue dans la chambre de traitement (2) au niveau de l'extrémité d'amenée du système de transport, **caractérisé en ce que**
- du liquide qui s'est accumulé dans la zone de collecte définie (7) de la chambre de traitement (2) est évacué dans un récipient collecteur (15) lorsqu'un niveau maximal de liquide est atteint,
- lorsque le niveau de liquide dans la chambre de traitement (2) atteint une valeur minimale prédéterminée, du liquide est ramené du récipient collecteur (15) dans la chambre de traitement (2),
- lorsque le niveau de liquide dans le récipient collecteur (15) atteint une valeur maximale prédéterminée, du liquide est ramené du récipient collecteur (15) dans la chambre de traitement (2) si le niveau maximal de liquide n'est pas atteint dans la chambre de traitement (2), et/ou est traité dans une autre unité de désinfection à haute température (27) destinée à du liquide pur.

2. Procédé selon la revendication 1, **caractérisé en ce que** le liquide du récipient collecteur (15), qui est à traiter dans l'unité de désinfection à haute température destiné à du liquide pur, passe dans un echangeur de chaleur (27) dans lequel il est au moins porté à une température et maintenu à cette température aussi longtemps que cela est nécessaire pour une désinfection ou une stérilisation.

3. Procédé selon la revendication 2, **caractérisé en ce que** la désinfection ou la stérilisation au moyen de l'échangeur de chaleur (27) est effectuée en continu, une surpression permanente étant engendrée dans l'échangeur de chaleur (27) par le fait que le flux de liquide traité sortant de l'échangeur de chaleur (27) est limité par un organe de restriction (31).

4. Procédé selon la revendication 2 ou 3, **caractérisé en ce que**, dans le cas où du liquide du récipient collecteur (15) n'est ramené dans la chambre de traitement (2) que lorsque le niveau de liquide dans la chambre de traitement (2) a atteint une valeur minimale prédéterminée, du liquide est évacué du récipient collecteur (15) par l'échangeur de chaleur (27) jusqu'à ce que le niveau de liquide dans le récipient collecteur (15) ait atteint une valeur moyenne prédéterminée.

5. Procédé selon la revendication 1, **caractérisé en ce que** le liquide à traiter dans l'autre unité de désinfection à haute température est désinfecté ou stérilisé dans le récipient collecteur (15), notamment au cours d'un processus de désinfection ou de stérilisation dans le récipient collecteur (15), et **en ce que** du liquide supplémentaire non traité est collecté dans un récipient tampon (33) qui est disposé entre la chambre de traitement (2) et le récipient collecteur (15).

6. Procédé selon l'une quelconque des revendications précédentes, **caractérisé en ce que** la zone de collecte définie (7) pour du liquide est créée par le fait que la chambre de traitement (2) est inclinée vers le haut dans le sens du transport.

7. Procédé selon l'une quelconque des revendications précédentes, **caractérisé en ce que** le système de transport comporte un transporteur à vis.

8. Dispositif destiné à la mise en oeuvre du procédé de traitement de substances contaminées, notamment infectées, selon les revendications 1 à 7, dans lequel celles-ci sont amenées par l'intermédiaire d'une unité d'entrée (4) à un système de transport s'étendant dans une chambre de traitement (2), chauffées et désinfectées ou stérilisées, ainsi qu'évacuées au niveau d'une sortie, une zone de collecte définie (7) pour du liquide étant prévue dans la chambre de traitement (2) au niveau de l'extrémité d'amenée du système de transport, **caractérisé en ce que**, par l'intermédiaire d'au moins une conduite (13) de liquide, la zone de collecte (7) de la chambre de traitement (2) est reliée à un récipient collecteur (15) de liquide, de telle sorte que du liquide puisse être amené de la zone de collecte (7) de la chambre de traitement (2) dans le récipient collecteur (15), et ramené de ce dernier dans la chambre de traitement (2), un indicateur de liquide (8, 9) étant prévu pour la détection des niveaux de remplissage respectifs, et **en ce que** le récipient collecteur (15) de liquide est relié à une autre unité de désinfection à haute température (27) par l'intermédiaire d'une conduite (26) de liquide.

9. Dispositif selon la revendication 8, **caractérisé en ce que** l'autre unité de désinfection à haute température comporte un échangeur de chaleur (27)

10. Dispositif selon la revendication 9, **caractérisé en ce que** le récipient collecteur (15) de liquide est conçu en tant qu'autre unité de désinfection à haute température.

11. Dispositif selon la revendication 10, **caractérisé en ce qu'**un récipient tampon (33) destiné au liquide provenant de la zone de collecte (7) de la chambre de traitement (2) est disposé entre la chambre de traitement (2) et le récipient collecteur (15) de liquide.

12. Dispositif selon l'une quelconque des revendications 8 à 11, **caractérisé en ce que** le récipient collecteur (15) de liquide fonctionne selon le principe d'un réservoir atmosphérique.

13. Dispositif selon l'une quelconque des revendications 8 à 12, **caractérisé en ce que** la chambre de traitement (2) est orientée vers le haut dans le sens du transport.

14. Dispositif selon l'une quelconque des revendications 8 à 13, **caractérisé en ce que** le système de transport comporte un transporteur à vis.
